# EUROPEAN PATENT APPLICATION

(11) **EP 4 682 909 A1**
(43) Date of publication of application: **21.01.2026**
(21) Application number: 24769668.5
(22) Date of filing: 29.01.2024
(51) Int. Cl.: H01B 7/04

(54) **ELECTRODE LEAD WIRE TIP STRUCTURE, ELECTRODE LEAD WIRE, AND ELECTRODE LEAD WIRE CONVEYING SYSTEM**

(30) Priority: 13.03.2023 CN 202310236781
(71) Applicant: MicroPort Soaring CRM (Shanghai) Co., Ltd., Shanghai 201203 (CN)
(72) Inventor: CHENG, Zhijun, Shanghai 201203 (CN); NING, Yunfeng, Shanghai 201203 (CN); SUN, Jiangkai, Shanghai 201203 (CN); ZHU, Xiaoming, Shanghai 201203 (CN); YU, Peng, Shanghai 201203 (CN)
(74) Representative: Lavoix
(86) International application number: PCT/CN2024/074365
(87) International publication number: WO 2024/187972

(57) **Abstract**

The present invention provides an electrode lead tip structure, an electrode lead and an electrode lead conveying system. The electrode lead tip structure includes a first support member, a flexible transition member, a second support member, a fixation member and an actuation and electrical connection member. The first support member, the flexible transition member, the second support member and the fixation member are connected in sequence in a direction, in which the electrode lead tip structure extends from a proximal end to a distal end thereof, and together define a cavity. The actuation and electrical connection member is at least partially received in the cavity, and the flexible transition member is configured to be bendable under the action of an external force applied thereto and able to restore its original shape after the external force is removed. According to the present invention, the electrode lead tip structure can be manipulated by an operator to be more easily advanced through a delivery sheath, reducing the time required to complete a surgical procedure, lowering the risk of patient infection, enabling the procedure to proceed smoothly and improving the success rate of surgery.

## Description

### TECHNICAL FIELD

The present invention relates to the field of medical devices and, in particular to, a tip structure of an electrode lead, an electrode lead and an electrode lead conveying system.

### BACKGROUND

Tip structures of existing electrode leads are long, stiff and unbendable. Consequently, such an electrode lead would encounter significant resistance when advanced through an associated delivery sheath, which may cause damage to the electrode lead, or even could not pass through the delivery sheath at all. It may be even more challenging for the electrode lead to pass through the delivery sheath, particularly when its distal end is designed for active helical fixation. On the other hand, an electrode lead, when designed with a freely flexible electrode lead tip structure for active helical fixation, may experience deformation of the electrode lead tip structure due to its freely flexible nature, and hence failure to effectively transfer a torque and proneness to damage to the electrode lead. Therefore, whether with a stiff or flexible electrode lead tip structure, such an electrode lead will be subject to significant push forces and torques during an implantation procedure, which not only tend to cause damage to the electrode lead, but also lead to difficulties in properly completing the procedure.

It should be noted that the information disclosed in this Background section is merely intended to provide a better understanding of the general context of the present invention and should not be taken as an acknowledgement or any form of admission that the information forms part of the common general knowledge of those skilled in the art.

### SUMMARY

In view of the problems of difficult advancement through a hollow delivery sheath by means of an operator's manipulation and proneness to electrode lead damage with tip structures of conventional electrode leads, there are provided herein a tip structure of an electrode lead, an electrode lead and an electrode lead conveying system. With the present invention, an operator's manipulation allows for easier advancement of the electrode lead tip structure through a delivery sheath, reducing the time required to complete a surgical procedure, lowering the risk of patient infection, enabling the procedure to proceed smoothly and increasing the success of the procedure.

To this end, in one aspect, there is provided herein a tip structure of an electrode lead, which includes a first support member, a flexible transition member, a second support member, a fixation member and an actuation and electrical connection member.

The first support member, the flexible transition member, the second support member and the fixation member are connected in sequence in a direction, in which the electrode lead tip structure extends from a proximal end to a distal end thereof, and together define a cavity extending through at its opposite ends respectively through the proximal and distal ends of the electrode lead tip structure.

The actuation and electrical connection member is at least partially received in the cavity.

The first support member includes a stiff hollow insulating structure for passage of the actuation and electrical connection member therethrough.

The flexible transition member is configured to be bendable under the action of an external force applied thereto and able to restore its original shape after the external force is removed.

Optionally, the first support member, the flexible transition member, the second support member and the fixation member may be hollow cylinders or hollow quasi-cylinders with collinear central axes.

Optionally, the electrode lead tip structure may have a diameter ranging from 1 mm to 3 mm and a length ranging from 5 mm to 50 mm.

Optionally, the first support member may further include a ring electrode disposed over an outer circumference of the stiff hollow insulating structure, wherein the stiff hollow insulating structure of the first support member includes a first insulating element and a first supporting element, which are both ring-shaped; the first insulating element is situated between the ring electrode and the first supporting element; the ring electrode, the first insulating element and the first supporting element are coaxially attached together in sequence; and the ring electrode is coupled to the flexible transition member.

Optionally, the flexible transition member may include a flexible transition tube and at least one connecting wire, the flexible transition tube including coupling hole(s), each corresponding to a respective one of the respective connecting wire(s) and extending through a tubular body of the flexible transition tube along an axis thereof, wherein:
each connecting wire has a length greater than a length of the flexible transition tube and is inserted through the respective coupling hole, and at least one of the connecting wire(s) is proximally fixedly attached to the ring electrode and distally coupled to the second support member; and
the flexible transition tube and the connecting wire(s) are configured to be bendable under the action of an external force applied thereto and able to restore their original shapes after the external force is removed.

Optionally, there may be at least two coupling holes evenly spaced circumferentially around the tubular body of the flexible transition tube. Additionally or alternatively, each coupling hole may have a shape selected from a circular cylinder, a triangular prism and a polygonal prism.

Optionally, each coupling hole may be shaped as a circular cylinder with a diameter ranging from 0.02 mm to 0.3 mm, or as a triangular prism with a cross-sectional side length ranging from 0.02 mm to 0.3 mm, or as a polygonal prism with a cross-sectional diagonal length ranging from 0.02 mm to 0.3 mm.

Optionally, when the external force is applied to the flexible transition tube and the connecting wire(s), the flexible transition tube may be able to bend under the action of the external force so as to have a central angle in the range of 45° to 180°.

Optionally, the flexible transition tube may have an outer diameter in the range of 1.5 mm to 3 mm and a length in the range of 2 mm to 20 mm.

Optionally, the second support member may include a second insulating element and a second supporting element, which are fixedly attached together coaxially and are both ring-shaped, and is provided as a stiff hollow insulating structure, the second supporting element disposed over an outer circumference of the second insulating element and fixedly coupled to the flexible transition member.

Optionally, the actuation and electrical connection member may include an actuator and a telescopic needle, the actuator disposed in the cavity and distally coupled to a proximal end of the telescopic needle,
the actuator configured to actuate the telescopic needle to move in the direction in which the electrode lead tip structure extends.

Optionally, the fixation member may include a fixation head and a fixation helix,
the fixation head proximally fixed to a distal end of the second support member, the fixation helix fixed at a distal end of the fixation head and extending away from the second support member,
wherein the telescopic needle extends through the fixation head and is able to be actuated by the actuator to distally move from the fixation head to a location beyond a distal end of the fixation helix, and the central axes of the telescopic needle, the fixation head and the fixation helix are collinear.

Optionally, the actuation and electrical connection member may further include a connector connecting the actuator and the telescopic needle, the connector located in the cavity corresponding to the second support member and able to move on an inner wall of the second support member.

To the above end, the present invention also provides an electrode lead including a coupling end, a lead body and the electrode lead tip structure as defined above, which are joined in sequence in a direction, in which the electrode lead extends from a proximal end to a distal end thereof.

To the above end, the present invention also provides an electrode lead conveying system including a delivery sheath and the electrode lead as defined above.

The delivery sheath includes a sheath entrance, a tubular body and a sheath exit, the tubular body including at least one bend.

The delivery sheath is configured to deliver the electrode lead to a target position within a target organic tissue.

Compared to the prior art, the electrode lead tip structure, electrode lead and conveying system of the present invention offer the following benefits:
The electrode lead tip structure includes a first support member, a flexible transition member, a second support member, a fixation member and an actuation and electrical connection member. The first support member, the flexible transition member, the second support member and the fixation member are connected in sequence in a direction, in which the electrode lead tip structure extends from a proximal end to a distal end thereof, and together defining a cavity extending through at its opposite ends respectively through the proximal and distal ends of the electrode lead tip structure. The actuation and electrical connection member is at least partially received in the cavity. The first support member includes a stiff hollow insulating structure for passage of the actuation and electrical connection member therethrough. The flexible transition member is configured to be bendable under the action of an external force applied thereto and able to restore its original shape after the external force is removed. Therefore, the electrode lead tip structure is a multi-segment structure with varying flexibility/stiffness, in which both the first and second support members are stiff and the flexible transition member is bendable under the action of an external force. With this arrangement, during operation of an operator for advancing the electrode lead tip structure through a curved delivery sheath, as required for implantation of the electrode lead, the bendability of the flexible transition member can reduce resistance from the delivery sheath to the electrode lead, thus facilitating passage of the electrode lead through the curved delivery sheath. Further, the first support member, the flexible transition member and the second support member can provide sufficient twist resistance, rigid support and bendability required for implantation of the electrode lead by a screwing operation. Thus, the electrode lead tip structure is simple in structure and overcomes the problem of difficult passage through a curved sheath associated with conventional stiff electrode lead tip structures and the problem of insufficient twist resistance and support due to easy deformability associated with conventional flexible electrode lead tip structures. It can be manipulated by an operator to be more easily advanced through a delivery sheath, thereby reducing the time required to complete a surgical procedure, lowering the risk of patient infection, enabling the procedure to proceed smoothly and increasing the success of the procedure.

As the electrode lead and conveying system of the present invention are based on the same inventive concept as the electrode lead tip structure of the present invention and thus has at least all the advantages of the latter, the advantages thereof are not repeated herein.

### BRIEF DESCRIPTION OF THE DRAWINGS

Fig. 1 is a schematic diagram showing the structure of an electrode lead according to a first embodiment of the present invention.
Fig. 2 is a schematic diagram showing the structure of a delivery sheath in an electrode lead conveying system according to a second embodiment of the present invention.
Fig. 3 is a schematic diagram showing the overall structure of an electrode lead tip structure according to a third embodiment of the present invention.
Fig. 4A is a schematic diagram showing the overall structure of a two-segment flexible transition tube of a flexible transition member in the electrode lead tip structure according to an implementation of the third embodiment of the present invention.
Fig. 4B is a schematic diagram showing the structure of the two-segment flexible transition tube of Fig. 4A inserted therein with connecting wires.
Fig. 4C is a front view of Fig. 4A in a curved configuration.
Fig. 5A schematically illustrates how a ring electrode, connecting wires, a second supporting element and a fixation member in the electrode lead tip structure are coupled in a rest configuration in accordance with an implementation of the embodiments of the present invention.
Fig. 5B schematically illustrates the coupled components of Fig. 5A in a bent configuration in which they are subject to an external force.

### List of Reference Numerals

100 coupling end; 200, lead body; 300, electrode lead tip structure;
310, first support member; 311, hollow insulating structure; 3111, first insulating element; 3112, first supporting element; 312, ring electrode;
320, flexible transition member; 321, flexible transition tube; 322, connecting wire; 323, coupling hole;
330, second support member; 331, second insulating element; 332, second supporting element;
340, fixation member; 341, fixation head; 342, fixation helix;
350, actuation and electrical connection member; 351, actuating helix; 352, telescopic needle; 353, connector;
410, sheath entrance; 420, first bend; 430, second bend; 440, sheath exit;
α, central angle.

### DETAILED DESCRIPTION

Objects, advantages and features of the present invention will become more apparent upon reading the following more detailed description of electrode lead tip structures, electrode leads and conveying systems proposed herein. Note that the figures are provided in a very simplified form not necessarily drawn to exact scale for the only purpose of helping to explain the disclosed embodiments in a more convenient and clearer way. It will be understood that the figures may not necessarily illustrate the structures described herein to scale and that illustrative features depicted in the figures to explain certain principles of the present invention may be slightly simplified. The specific design features of the invention as disclosed herein, including, for example, specific dimensions, orientations, locations and shapes, will be determined in part by the particular intended application and use environment. Additionally, in the embodiments described below, the same reference numerals may be sometimes used throughout different figures to indicate identical components or components with identical functions, while description thereof may not be repeated. In this specification, similar reference numerals and letters refer to similar items in the figures, and thus once an item is defined in one figure, it may not be discussed for following figures.

Where appropriate, the terms so used are interchangeable. Likewise, if a method is described herein as comprising a series of steps, the order of these steps as presented herein is not necessarily the only order in which they can be performed, and some of the stated steps may be omitted and/or other steps not described herein may be added to the method.

It is a principal object of the present invention to provide an electrode lead tip structure, an electrode lead and an electrode lead conveying system. With the present invention, an operator's manipulation allows for easier advancement of the electrode lead tip structure through a delivery sheath, reducing the time required to complete a surgical procedure, lowering the risk of patient infection, enabling the procedure to proceed smoothly and increasing the success of the procedure.

Electrode leads, delivery sheaths and electrode lead tip structures according to embodiments of the present invention, which attain the above object, are described in sequence below to provide a better understanding of the invention. In addition, as used herein, the term "proximal" refers to an end closer to an operator (i.e., farther away from a lesion or target organic tissue), and "distal" refers to an end farther away from the operator (i.e., closer to the lesion or target organic tissue). Further, as would be appreciated by those skilled in the art, the present invention is not limited to any particular application of the electrode lead, and possible applications may include, but are not limited to, cardiac pacing, therapeutic ablation and physiological signal acquisition.

### Example 1

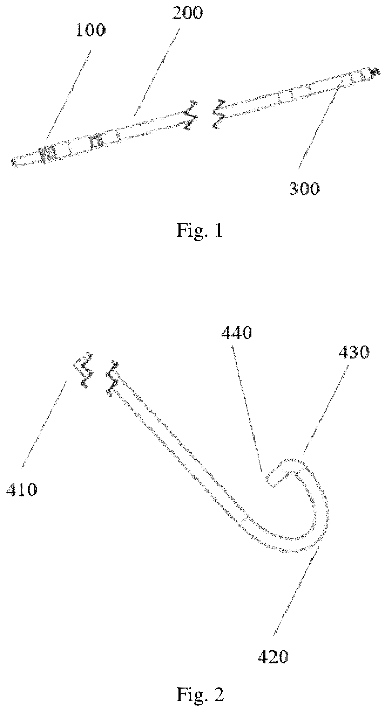

In a first embodiment of the present invention, there is provided an electrode lead. Particular reference is made to Fig. 1, which schematically illustrates a structural diagram of the electrode lead according to one embodiment of this embodiment. As can be seen from Fig. 1 and as would be appreciated by those skilled in the art, the electrode lead is simple in structure because it includes only a coupling end 100, a lead body 200 and an electrode lead tip structure 300. The coupling end 100, the lead body 200 and the electrode lead tip structure 300 are joined in sequence in a direction in which the electrode lead extends from its proximal end to its distal end. More specifically, there are many possible implementations for the electrode lead tip structure 300, as described in greater detail below. For more details of these electrode lead tip structures 300, reference can be made to the following description, and detailed description thereof is omitted here for the sake of brevity.

Further, since the electrode lead provided by this embodiment is based on the same inventive concept as the electrode lead tip structures 300 described in the following embodiments, the electrode lead tip structures 300 can also be manipulated by an operator to be more easily advanced through a delivery sheath, thereby reducing the time required to complete a surgical procedure, lowering the risk of patient infection, enabling the procedure to proceed smoothly and increasing the success of the procedure. In other words, the electrode lead of this embodiment has at least all the advantages of the electrode lead tip structures 300 described below. For more information about the advantages of those electrode lead tip structures 300, reference can be made to the following description, and detailed description thereof is omitted here for the sake of brevity.

### Example 2

In a second embodiment, there is provided an electrode lead conveying system, which includes a delivery sheath and an electrode lead. The delivery sheath is used to deliver the electrode lead to a target position within a target organic tissue. Below, the delivery sheath in the electrode lead conveying system of this embodiment will be described in detail, and for more details of the electrode lead in the electrode lead conveying system, reference can be made to the above description in connection with the first embodiment. For the sake of brevity, detailed description of the electrode lead is not repeated here.

In order to provide a better understanding of this embodiment, before the delivery sheath of this embodiment is described in detail, it should be noted that, and as would be appreciated by those skilled in the art, the delivery sheath may adopt a curved design in some applications, depending on patients' anatomies. In these scenarios, if the electrode lead has a flexible or stiff electrode lead tip structure, when advanced through the curved delivery sheath, it may suffer from the problem of non-crossability or proneness to damage, as described in the Background section.

For example, reference is made to Fig. 2, which shows a schematic structural view of the delivery sheath in the electrode lead conveying system according to the second embodiment. As can be seen from Fig. 2, the delivery sheath in the electrode lead conveying system of this embodiment includes a sheath entrance 410, a tubular body (not shown) and a sheath exit 440. The tubular body includes at least one bend. With this arrangement, the delivery sheath in the electrode lead conveying system of this embodiment can be suitably used in more applications with different anatomic structures of target organ tissue.

With continued reference to Fig. 2, in one preferred exemplary implementation, the delivery sheath includes one first bend 420 and one second bend 430, both of the first bend 420 and the second bend 430 are close to a distal end of the delivery sheath. Additionally, the first bend 420 (referred to as a large bend) has a first bending angle 510 greater than a first bending angle 510 of the second bend 430 (referred to as a small bend). Further, the second bend 430 is closer to the distal end of the delivery sheath than the first bend 420. Furthermore, both the first bend 420 and the second bend 430 are both counterclockwise bends (as viewed in the orientation of the delivery sheath shown in Fig. 2) so that a distal portion of the delivery sheath resembles a fishhook. Thus, this "fishhook-shaped" delivery sheath can change its direction of advancement twice in target organic tissue, facilitating a surgeon's operation.

In some other preferred implementations, the bending angles of the first bend 420 and the second bend 430 are changeable, making the delivery sheath steerable. This enables an operator (e.g., a surgeon) to more easily curve the distal end of the delivery sheath at different desired angles within the body of a patient by performing corresponding adjustment operations outside the patient's body, thereby further increasing the suitability for use in applications with different anatomic structures of target organ tissue.

It should be noted that, and as would be appreciated by those skilled in the art, the present invention is not limited to any particular number of bends of the delivery sheath, or to any particular location, bending angle or direction of any of these bends. In practical use, any appropriate scheme may be selected as desired. It will be understood that the bending angles of the bends of the delivery sheath and distance(s) between adjacent bends are preferably determined in accordance with a length and bendability (corresponding to a maximum central angle of the flexible transition member 320 when it is stressed) of a flexible transition member 320 in an electrode lead tip structure 300. This can better protect the electrode lead, facilitate the operator's manipulation for advancing the electrode lead tip structure 300 through the delivery sheath, reduce the time required to complete a surgical procedure, make the procedure more efficient and reduce patient trauma.

### Example 3

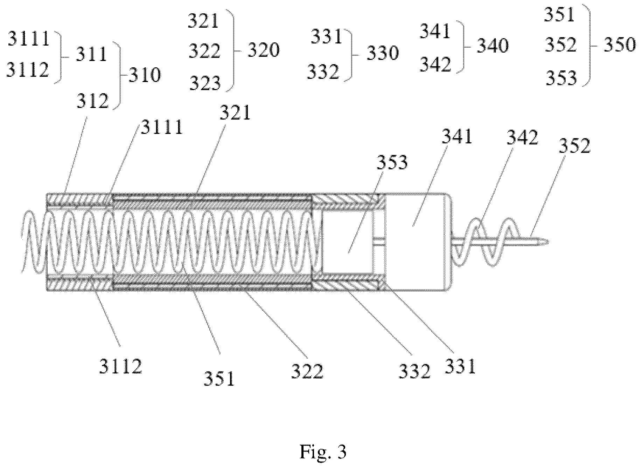

In a third embodiment, there is provided an electrode lead tip structure 300. Particular reference is made to Fig. 3, which is a schematic diagram showing the overall structure of the electrode lead tip structure of this embodiment. As can be seen from Fig. 3, the electrode lead tip structure 300 of this embodiment includes a first support member 310, a flexible transition member 320, a second support member 330, a fixation member 340 and an actuation and electrical connection member 350. More specifically, in a direction in which the electrode lead tip structure 300 extends from its proximal end (on the left, as shown) to its distal end (on the right, as shown), the first support member 310, the flexible transition member 320, the second support member 330 and the fixation member 340 are fixedly connected in sequence and together define a cavity (not shown). The cavity extends through the electrode lead tip structure 300, with its opposite ends being respectively located at proximal and distal ends of the latter. The actuation and electrical connection member 350 is at least partially received in the cavity. The first support member 310 includes a stiff hollow insulating structure 311, and the flexible transition member 320 is configured to be bendable under the action of an external force and able to restore its original shape after the external force is removed.

Therefore, the electrode lead tip structure 300 provided in this embodiment includes the first support member 310, the flexible transition member 320 and the second support member 330. The first support member 310 is stiff, the flexible transition member 320 is bendable under the action of an external force, and the second support member 330 is stiff. With the multi-segment stiff-flexible hybrid arrangement, during operation of an operator for advancing the electrode lead tip structure 300 through a curved delivery sheath, as required for implantation of the electrode lead, the flexible transition member 320 deforms under applied forces, thereby reducing resistance from the delivery sheath to the electrode lead, thus facilitating passage of the electrode lead through the curved delivery sheath. Further, the first support member 310, the flexible transition member 320 and the second support member 330 of the electrode lead tip structure 300 of this embodiment can provide sufficient twist resistance, rigid support and bendability required for implantation of the electrode lead by a screwing operation. Thus, the electrode lead tip structure 300 of this embodiment is simple in structure and overcomes the problem of difficult passage through a curved sheath associated with conventional stiff electrode lead tip structures and the problem of insufficient twist resistance and support due to easy deformability associated with conventional flexible electrode lead tip structures. The electrode lead tip structure 300 of this embodiment can be manipulated by an operator to be more easily advanced through a delivery sheath, thereby reducing the time required to complete a surgical procedure, lowering the risk of patient infection, enabling the procedure to proceed smoothly and increasing the success of the procedure.

In some exemplary implementations, the actuation and electrical connection member 350 includes an actuator and a telescopic needle. The actuator is disposed in the cavity, with its distal end being coupled to a proximal end of the telescopic needle 352. The actuator is configured to actuate the telescopic needle 352 in the direction of the extension of the electrode lead tip structure (i.e., to the left or right, as viewed in the orientation of Fig. 3). Preferably, the part of the actuation and electrical connection member 350 received in the cavity includes the actuator and part of the telescopic needle 352, and the rest of the telescopic needle 352 is located outside the cavity. More specifically, as would be appreciated by those skilled in the art, the present invention is not limited to any particular form of the actuator. Preferably, the actuator is an actuating helix 351, and there is an internal thread in an inner wall of the cavity in engagement with the actuating helix 351. When the actuating helix 351 is rotated about its own axis in the internal thread, the actuating helix 351 moves in the direction of extension of the electrode lead tip structure (i.e., to the left or right, as viewed in the orientation of Fig. 3) and causes the telescopic needle 352 in synchronization therewith.

Preferably, with continued reference to Fig. 3, the first support member 310, the flexible transition member 320, the second support member 330 and the fixation member 340 are hollow cylinders or hollow quasi-cylinders with collinear central axes (when the flexible transition member 320 is at rest, i.e., without any external force acting on the flexible transition member 320). Such cylindrical or quasi-cylindrical shapes of the first support member 310, the flexible transition member 320, the second support member 330 and the fixation member 340 can facilitate delivery of an electrode lead with the electrode lead tip structure 300 of the present embodiment through a delivery sheath to a target position within a target organic tissue. Meanwhile, the cavity defined by the first support member 310, the flexible transition member 320, the second support member 330 and the fixation member 340 may also be cylindrical and have a central axis collinear with the central axes of them, allowing the actuator to more easily actuate the telescopic needle 360 to move in the direction of extension of the electrode lead tip structure 300. Further, central axes of the actuator and of the telescopic needle 360 may also be collinear with the central axes of the first support member 310, the flexible transition member 320, the second support member 330 and the fixation member 340, allowing the actuator to even more easily drive the telescopic needle 360 to move in the direction of extension of the electrode lead tip structure 300.

It should be noted that, and as would be appreciated by those skilled in the art, although the first support member 310, the flexible transition member 320, the second support member 330 and the fixation member 340 have been described above as being preferably provided as hollow cylinders or hollow quasi-cylinders with collinear central axes, the present invention is not so limited. In alternative implementations, the first support member 310, the flexible transition member 320, the second support member 330 and the fixation member 340 may not be cylinders, but polygonal prisms, elliptical cylinder or the like, for example.

Preferably, the electrode lead tip structure 300 has a diameter (outer diameter) in the range of 1 mm to 3 mm, the electrode lead tip structure 300 has a length in the range of 5 mm to 50 mm. An outer diameter of the electrode lead tip structure 300 range from 1 mm to 3 mm, the outer diameter of the electrode lead tip structure 300 exceeding the 3-mm upper limit of the above outer diameter range may create difficulties in its crossing through a delivery sheath. On the other hand, an outer diameter of the electrode lead tip structure 300 below the 1-mm lower limit of said range may lead to a size of the its cavity, which is too small to allow the actuator to cause desirable reciprocation of the telescopic needle along the direction of extension of the electrode lead tip structure 300. The aforementioned length range of 5 mm to 50 mm for the electrode lead tip structure 300 enables the first support member 310, the flexible transition member 320, the second support member 330 and the fixation member 340 to have appropriate lengths, which ensure that the electrode lead tip structure 300 exhibit both desirable stiffness and good flexibility. A length of the electrode lead tip structure 300 below the lower limit of the length range may spoil the advantage of both desirable stiffness and good flexibility of the electrode lead tip structure 300. On the other hand, a length above the upper limit of said range may fail to enable the electrode lead tip structure 300 to be advanced through a delivery catheter with dense, contiguous bends and may cause damage to the electrode lead in the process of advancement.

Preferably, with continued reference to Fig. 3, the first support member 310 further includes a ring electrode 312 disposed over an outer circumference of the stiff hollow insulating structure 311. The stiff hollow insulating structure 311 of the first support member 310 includes a first insulating element 3111 and a first supporting element 3112, both of the first insulating element 3111 and the first supporting element 3112 are in the shape of rings. The first insulating element 3111 is situated between the ring electrode 312 and the first supporting element 3112. The ring electrode 312, the first insulating element 3111 and the first supporting element 3112 are fixedly attached together so as to be coaxial. The ring electrode 312 is coupled to the flexible transition member 320. With this arrangement, the first insulating element 3111 isolates the actuating helix 351 from the ring electrode 312, avoiding the outer ring electrode 312 from being electrically connected to the inner telescopic needle 352 (the telescopic needle 352 is conductive, i.e., made of a conductive material, and used as an electrode), i.e., insulating them from each other. The first supporting element 3112 is a stiff hollow insulating structure capable of providing effective support to the first insulating element 3111 and the ring electrode 312. In this way, good proximal insulation and stiffness of the electrode lead tip structure 300 can be assured.

In a particularly preferred implementation, the ring electrode 312, the first insulating element 3111 and the first supporting element 3112 are tightly and coaxially fixed together. Additionally, the ring electrode 312 may be fixedly coupled to the flexible transition member 320 in a seamless manner. In this way, this mutually nested design additionally assures proximal stiffness of the electrode lead tip structure 300, facilitating the placement of the actuator in the cavity together with the telescopic needle 352.

It should be noted that, and as would be appreciated by those skilled in the art, although the ring electrode 312, the first insulating element 3111 and the first supporting element 3112 have been described above as being tightly and coaxially fixed together, as an example, the present invention is not so limited. In addition, the first insulating element 3111 is preferably made of, among others, silicone, polyurethane or polypropylene, and the first supporting element 3112 is preferably made of, among others, polyvinyl chloride (PVC), polyether ether ketone (PEEK) or polyethylene.

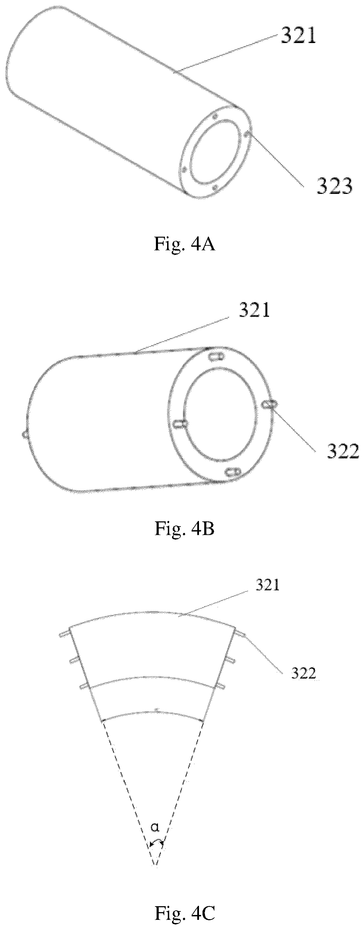

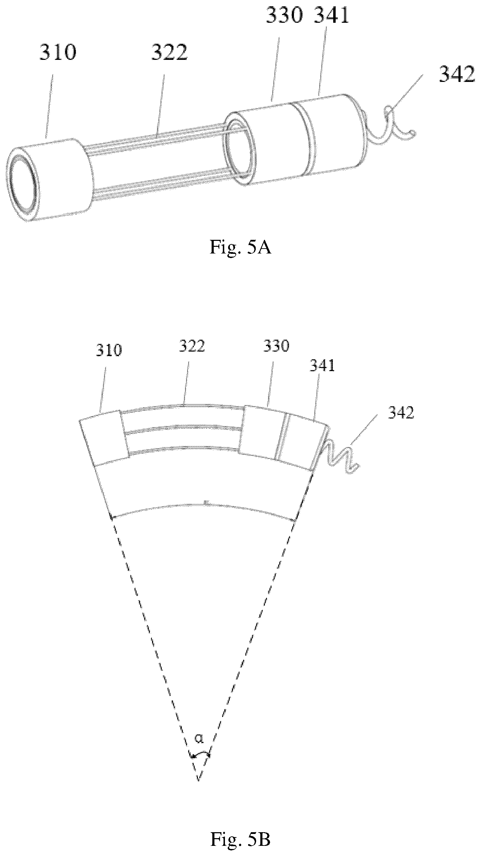

Preferred implementations are described below with reference to Figs. 4A to 4C and 5A to 5B. Fig. 4A is a schematic diagram showing the overall structure of a two-segment flexible transition tube 321 in the flexible transition member 320 of the electrode lead tip structure 300 according to an implementation of the third embodiment of the present invention. Fig. 4B is a schematic diagram showing the structure of the two-segment flexible transition tube 321 of Fig. 4A inserted therein with connecting wires 322. Fig. 4C is a front view of Fig. 4A in a curved configuration. Fig. 5A schematically illustrates how the ring electrode 312, the connecting wires 322 (the flexible transition tube 321 is not shown for clarity), a second supporting element 332 and the fixation member 340 in the electrode lead tip structure 300 are coupled in a rest configuration in accordance with an implementation of the embodiments of the present invention. Fig. 5B schematically illustrates the coupled components of Fig. 5A in a bent configuration in which they are subject to an external force. As can be seen from Figs. 4A to 4C, the flexible transition member 320 includes a flexible transition tube 321 and at least one connecting wire 322. The flexible transition tube 321 has coupling hole(s) 323 each corresponding to a respective one of the connecting wire(s) 322. Each coupling hole 323 extends through a tubular body of flexible transition tube 321 along an axis thereof, and each connecting wire 322 has a length greater than that of the flexible transition tube 321. The connecting wire(s) 322 is/are inserted in the respective coupling hole(s) 323, and at least one of the connecting wire(s) 322 is proximally fixed to the ring electrode 312 and distally coupled to the second support member 330 (e.g., to a fixation head 341 of the fixation member 340). The flexible transition tube 321 and the connecting wire(s) 322 are configured to be bendable under the action of an external force and to be able to return to the original shapes after the external force is removed. With this arrangement, the stiffness of the ring electrode 312 (more precisely, of the mutually nested and tightly fixed outer ring electrode 312, intermediate first insulating element 3111 and inner first supporting element 3112), the limited flexibility of the connecting wire(s) 322 inserted in the respective coupling hole(s) 323 in the flexible transition tube 321 and the stiffness of the second support member 330 (e.g., of a connector 353 of the second support member 330) entail a design with both desirable stiffness and good flexibility, which enables the electrode lead tip structure 300 to exhibit appropriate degrees of twist resistance, stiffness and bendability. Thus, an operator can manipulate the electrode lead tip structure 300 to more easily advance the electrode lead through a delivery sheath with a reduced risk of damage to the electrode lead tip structure 300.

It should be noted that, and as would be appreciated by those skilled in the art, the present invention is not limited to any particular material of the flexible transition tube 321 or the connecting wire(s) 322. The flexible transition tube 321 may be made of an elastic soft material, which makes the flexible transition tube 321 bendable under the action of an external force. Moreover, in the case of the connecting wire(s) 322 being inserted, the connecting wire(s) 322 is/are also bendable under the action of an external force and able to restore its/their original shape(s) after the external force is removed, together with the flexible transition tube 321. The flexible transition tube 321 is preferably made of silicone or polyurethane.

In one exemplary implementation, there are at least two coupling holes 323 (and hence the same number of respective connecting wires 322; this description is not repeated below). The multiple coupling holes 323 are evenly spaced circumferentially around the tubular body of the flexible transition tube 321. Preferably, examples of the shape of the coupling holes 323 may include circular cylinders, triangular prisms and polygonal prisms. Evenly arranging the coupling holes 323 circumferentially around the tubular body of the flexible transition tube 321 enables the ring electrode 312, the flexible transition tube 321 and the second support member 330 to be more uniformly stressed during bending of the flexible transition tube 321, thus allowing the electrode lead tip structure 300 to be bent without being twisted at all. Additionally, compared with the case of a single coupling hole 323 in the flexible transition tube 321, arranging multiple coupling holes 323 therein can result in enhanced stability of the flexible transition tube 321. Implementing the coupling holes 323 as circular cylinders, triangular prisms or polygonal prisms allows for easier insertion of the connecting wires 322 therein. It should be noted that, the present invention is not limited to any particular number of coupling holes 323 or any particular number of connecting wires 322. The number of connecting wires 322 may be greater than that of coupling holes 323, and in this case, for example, in each coupling hole 323, one or more connecting wires 322 may be disposed. Preferably, the number of connecting wire 322 is equal to that of coupling holes 323. For example, in this embodiment, four connecting wires 322 and four coupling holes 323 are included.

In some exemplary implementations, examples of the shape of the coupling holes 323 include cylinders with a diameter ranging from 0.02 mm to 0.3 mm, triangular prisms with a cross-sectional side length ranging from 0.02 mm to 0.3 mm and polygonal prisms with a cross-sectional diagonal length ranging from 0.02 mm to 0.3 mm. This enables easy insertion of the connecting wires 322 without adversely affecting the ruggedness or flexibility of the flexible transition tube 321.

Referring to Fig. 4C, in one exemplary implementation, when the flexible transition tube 321 and the connecting wires 322 are subject to an external force, the flexible transition tube 321 is bendable under the action of the external force so as to have a central angle α in the range of 45° to 180° (i.e., 45°≤α≤180°). That is, the flexible transition tube 321 may be configured to bend between a non-deflected configuration (where the longitudinal axis connecting the distal and proximal ends of the flexible transition tube 321 forms a 180° angle, indicating complete linear alignment) and a maximally bent configuration (where a central angle of 45° of the flexible transition tube 321, i.e., it distally and proximally extends in directions intersecting at an angle of 45°). This enables the electrode lead to be smoothly advanced through a curved delivery sheath while not affecting the ability of the actuation and electrical connection member 350 to move forth and back within the cavity.

In one exemplary implementation, the flexible transition tube 321 has an outer diameter in the range of 1.5 mm to 3 mm, and the flexible transition tube 321 has a length in the range of 2 mm to 20 mm.

It should be noted that, and as would be appreciated by those skilled in the art, the dimensions of the coupling holes 323 and the first bending angle 510, at which the flexible transition tube 321 and the connecting wires 322 are bendable under the action of an external force, may be properly determined as desired in practical applications, and the present invention is not limited in this regard.

With continued reference to Fig. 3, in a preferred implementation, the second support member 330 includes a second insulating element 331 and a second supporting element 332, which are fixedly attached together so as to be coaxial with each other. Both the second insulating element 331 and the second supporting element 332 are ring-shaped, and the second support member 330 is a stiff hollow insulating structure. The second supporting element 332 is disposed over an outer circumference of the second insulating element 331, and the second supporting element 332 is fixedly coupled to the flexible transition member 320. This arrangement assures good insulation and stiffness of the electrode lead tip structure 300. Additionally, the second insulating element 331 and the second supporting element 332 may be coaxially attached together in a seamless manner. It should be noted that, although the second insulating element 331 and the second supporting element 332 have been described above as being coaxially attached together in a seamless manner, as an example, the present invention is not so limited. The second insulating element 331 is preferably made of silicone, polyurethane or polypropylene, and the second supporting element 332 is preferably made of PVC, PEEK or polyethylene.

More specifically, the distal ends of several of the connecting wires 322 are fixedly connected to a proximal end of the second supporting element 332, and correspondingly, the proximal end of the connecting wire 322 fixedly connected to the proximal end of the second supporting member 332 is fixedly connected to the ring electrode 312. It should be noted that, as described above, the telescopic needle 352 is made of a conductive material and used as an electrode. Preferably, the second insulating element 331 has an axial cross-sectional shape resembling the letter "L", which has been rotated into a horizontal orientation. In particular, the second insulating element 331 may have a first portion, which extends in its own direction of extension (e.g., horizontally as viewed in the orientation of Fig. 3) and corresponds to the long arm of the letter "L", and a second portion, which extends perpendicularly to the direction of extension (e.g., vertically as viewed in the orientation of Fig. 3) and corresponds to the short arm of the letter "L". The first portion can prevent the telescopic needle 352 from being electrically connected to the ring electrode 312 by any of the connecting wires 322 in the flexible transition tube 321. The second portion can prevent the fixation member 340 from being electrically connected to the ring electrode 312 by any of the connecting wires 322 in the flexible transition tube 321.

With continued reference to Fig. 3, in one exemplary implementation, the fixation member 340 includes a fixation head 341 and a fixation helix 342. The fixation head 341 is proximally fixed to a distal end of the second support member 330 (more precisely, of the second insulating element 331 shown in Fig. 3), and the fixation helix 342 is fixed at a distal end of the fixation head 341. The telescopic needle 352 extends through both the fixation head 341 and the fixation helix 342 in such a manner that central axes of the telescopic needle 352, the fixation head 341 and the fixation helix 342 are collinear. This allows the telescopic needle 352 to be actuated by the actuating helix 351 to more easily penetrate into a target position within a target organic tissue, followed by manipulating the fixation helix 342 to anchor and fix the electrode lead at the target position. It should be noted that, the telescopic needle 352 is preferred to be a pointed metal needle extending along the central axis of the electrode lead tip structure 300. This allows it to be actuated by the actuating helix 351 to even more easily penetrate into the target position within the target organic tissue.

With continued reference to Fig. 3, in a preferred implementation, the electrode lead tip structure 300 further includes a connector 353 connecting the actuator to the telescopic needle 352. The connector 353 is disposed in the cavity corresponding to the second support member 330 so as to be able to slide on an inner wall of the second support member 330. The connector 353 provides a rugged connection between the actuator (e.g., the actuating helix 351) and the telescopic needle 352, which additionally ensures that the actuator and the telescopic needle 352 are ruggedly connected to each other. Moreover, it can guide axial movement of the telescopic needle 352. Further, since the connector 353 is disposed in the cavity corresponding to the stiff second support member 330, an operator can more easily cause the telescopic needle 352 to penetrate into a target position within a target organic tissue. Furthermore, the ability of the connector 353 to slide on the inner wall of the second support member 330 can assume stability of the telescopic needle 352 during movement.

In order to provide a better understanding of the present invention, a method of delivering an electrode lead tip structure 300 according to the invention through a delivery sheath according to the invention to target organic tissue is briefed below.

In one exemplary embodiment, an operator may first insert the lead body 200 and the electrode lead tip structure 300 of the electrode lead into the delivery sheath through the sheath entrance 410 and then advance the electrode lead successively through the first bend 420 and the second bend 430 until the electrode lead tip structure 300 is partially or entirely exposed outside of the sheath exit 440. As can be seen from Figs. 2 and 5B, since the delivery sheath (as would be appreciated by those skilled in the art, the delivery sheath is hollow and can accommodate the electrode lead therein) has a central axis, which is not straight but has multiple curved sections, and due to a relatively large length of the electrode lead tip structure 300, during the insertion and advancement of the electrode lead, the electrode lead tip structure 300 would be subject to significant reaction forces from an inner wall of the delivery sheath, which resist the passage of the electrode lead tip structure 300 through the delivery sheath and bend the flexible transition member 320 of the electrode lead tip structure 300. Thus, the passage through the delivery sheath continues at a first central angle α until the electrode lead tip structure 300 is exposed outside the sheath exit 440. Finally, the electrode lead is turned, concurrently with the assembly of the connecting wires 322, the ring electrode 312 and the second supporting element 332 providing axial support and twist resistance, screwing the fixation helix 342 into the target organic tissue (e.g., the myocardium). The actuating helix 351 is then turned to cause the telescopic needle 352 to penetrate into a target position (e.g., the left bundle branch) within the target organic tissue (e.g., the myocardium) until the electrode lead is successfully implanted and anchored to the target position within the target organic tissue. It should be particularly noted that, and as would be appreciated by those skilled in the art, the present invention is not limited to any particular effect provided by the penetration of the telescopic needle 352 of the electrode lead at the target position or by the electrode lead. In alternative embodiments, the electrode lead may also be anchored at other sites, such as to the ventricular septum or the atrial wall, to optionally sense physiological information from the target organic tissue or pace (stimulate) the target organic tissue to return it to normal function.

It will be understood that, as used herein, the terms "center", "longitudinal", "transverse", "length", "width", "thickness", "up", "down", "front", "rear", "left", "right", "vertical", "horizontal", "top", "bottom", "inner", "outer", "clockwise", "counterclockwise", "axial", "radial", "circumferential" and so on may be used herein to describe orientations or positional relationships as viewed in the annexed figures. They are for convenience and ease of description of the present invention only and do not indicate or imply that the described apparatus or element must assume, or be constructed or operated in, a particular orientation. Therefore, they are not to be construed as limiting the present invention.

In addition, when used herein, the terms "first", "second" and the like are intended only for illustration and are not to be construed as denoting or implying relative importance, or as implicitly indicating the number of the referenced items. Accordingly, defining an item with "first" or "second" is an explicit or implicit indication of the presence of one or at least two such items, unless the context clearly dictates otherwise. When used herein, the term "plurality" means "at least two", such as two or three, unless otherwise clearly defined.

As used herein, unless otherwise clearly specified or defined, the terms "mounted", "coupled", "connected", "secured" and variants thereof should be interpreted in a broad sense. For instance, a connection may be a permanent, detachable or integral connection, or a mechanical or electrical connection, or a direct or indirect connection with one or more intervening media, or an internal communication or interaction between two elements. Those of ordinary skill in the art can understand the specific meanings of the above-mentioned terms herein, depending on their context.

As used herein, unless otherwise clearly specified or defined, when a first feature is described as being "above" or "below" a second feature, it may be either in direct contact with the second feature, or in indirect contact with one or more intervening media being present therebetween. When a first feature is referred to as being "on", "above" or "on top of" a second feature, it may be right or obliquely on, above or on top of the second feature, or simply located at a higher level than the second feature. When a first feature is referred to as being "under", "below" or "at bottom of" a second feature, it may be right or obliquely under, below or at bottom of the second feature, or simply located at a lower level than the second feature.

Reference throughout this specification to "one embodiment", "some embodiments", "an example", "a specific example" "some examples" or the like means that a particular feature, structure, material, or characteristic described in connection with the embodiments or examples is included in at least one embodiment or example of the invention. Thus, the appearances of those phrases in various places throughout this specification are not necessarily referring to the same embodiment or example of the invention. Furthermore, the particular features, structures, materials, or characteristics may be combined in any suitable manner in any one or more embodiments. Further, should there be no contradiction, one of ordinary skill in the art can combine the various embodiments, examples and features thereof described herein in any combination.

Various electrode lead tip structure, electrode lead and electrode lead conveying system configurations disclosed herein have been described in detail above with respect to the foregoing embodiments. Of course, the above description is merely that of a few preferred modes of carrying out the invention and is in no way intended to limit the scope thereof. Possible configurations of the present invention include, but are not limited to, those described in the foregoing embodiments, and those skilled in the art can extrapolate other implementations in light of the above-described embodiments. Accordingly, any and all variations and modifications made by those of ordinary skill in the art to which the present invention pertains in light of the above teachings are intended to fall within the scope thereof as defined by the appended claims.

## Claims

1. An electrode lead tip structure, comprising a first support member, a flexible transition member, a second support member, a fixation member and an actuation and electrical connection member,
in an extension direction of the electrode lead tip structure from a proximal end to a distal end, the first support member, the flexible transition member, the second support member and the fixation member sequentially connected to define a cavity, opposite ends of the cavity respectively extending through the proximal end and the distal end of the electrode lead tip structure,
the actuation and electrical connection member at least partially received in the cavity,
wherein the first support member comprises a stiff hollow insulating structure for passage of the actuation and electrical connection member therethrough, and
the flexible transition member is configured to be bendable under the action of an external force applied thereto and able to restore its original shape after the external force is removed.

2. The electrode lead tip structure according to claim 1, wherein the first support member, the flexible transition member, the second support member and the fixation member are hollow cylinders or hollow quasi-cylinders with collinear central axes.

3. The electrode lead tip structure according to claim 1, wherein the first support member further comprises a ring electrode disposed over an outer circumference of the stiff hollow insulating structure, wherein the stiff hollow insulating structure of the first support member comprises a first insulating element and a first supporting element, which are both ring-shaped; the first insulating element is situated between the ring electrode and the first supporting element; the ring electrode, the first insulating element and the first supporting element are fixedly connected in sequence and coaxial with each other; and the ring electrode is connected to the flexible transition member.

4. The electrode lead tip structure according to claim 1, wherein: the flexible transition member comprises a flexible transition tube and at least one connecting wire, the flexible transition tube comprises at least one coupling hole, each corresponds to a respective connecting wire and extends through a tubular body of the flexible transition tube along an axis of the flexible transition tube,
each connecting wire has a length greater than a length of the flexible transition tube and is inserted through the respective coupling hole, and a proximal end of the at least one connecting wire is fixedly connected to the ring electrode, and a distal end of the at least one connecting wire fixedly connected to the ring electrode is connected to the second support member; and
the flexible transition tube and the at least one connecting wire are configured to be bendable under the action of an external force applied thereto and able to restore their original shapes after the external force is removed.

5. The electrode lead tip structure according to claim 4, wherein there are at least two coupling holes evenly spaced circumferentially around the tubular body of the flexible transition tube, and/or wherein each coupling hole has a shape selected from a circular cylinder, a triangular prism and a polygonal prism.

6. The electrode lead tip structure according to claim 5, wherein each of the at least two coupling hole is shaped as a circular cylinder with a diameter ranging from 0.02 mm to 0.3 mm, or as a triangular prism with a cross-sectional side length ranging from 0.02 mm to 0.3 mm, or as a polygonal prism with a cross-sectional diagonal length ranging from 0.02 mm to 0.3 mm.

7. The electrode lead tip structure according to claim 4, wherein when the external force is applied to the flexible transition tube and the at least one connecting wire, the flexible transition tube is able to bend under the action of the external force so as to have a central angle in the range of 45° to 180°.

8. The electrode lead tip structure according to claim 4, wherein the flexible transition tube has an outer diameter in the range of 1.5 mm to 3 mm, and wherein the flexible transition tube has a length in the range of 2 mm to 20 mm.

9. The electrode lead tip structure according to claim 1, wherein: the second support member comprises a second insulating element and a second supporting element fixedly connected coaxially, the second insulating element and the second supporting element are both ring-shaped, the second supporting element is provided as an insulating structure which is stiff and hollow, the second supporting element is disposed over an outer circumference of the second insulating element, and the second supporting element is fixedly connected to the flexible transition member.

10. The electrode lead tip structure according to claim 9, wherein the second insulating element and the second supporting element are fixedly connected coaxially in a seamless manner.

11. The electrode lead tip structure according to any one of claims 1 to 10, wherein the actuation and electrical connection member comprises an actuator and a telescopic needle, the actuator disposed in the cavity and a distal end of the actuator connected to a proximal end of the telescopic needle,
the actuator configured to actuate the telescopic needle to move in the extension direction of the electrode lead tip structure.

12. The electrode lead tip structure according to claim 11, wherein the fixation member comprises a fixation head and a fixation helix,
a proximal end of the fixation head fixedly connected to a distal end of the second support member, the fixation helix fixed at a distal end of the fixation head and extending away from the second support member,
wherein the telescopic needle extends through the fixation head, when actuated by the actuator, a distal end of the telescopic needle is able to move from the fixation head to a location beyond a distal end of the fixation helix, and the central axes of the telescopic needle, the fixation head and the fixation helix are collinear.

13. The electrode lead tip structure according to claim 11, wherein the actuation and electrical connection member further comprises a connector connecting the actuator and the telescopic needle, the connector located in the cavity corresponding to the second support member and able to slide along an inner wall of the second support member.

14. An electrode lead, comprising a coupling end, a lead body and the electrode lead tip structure of any one of claims 1 to 13, which are joined in sequence in the extension direction of the electrode lead tip structure from the proximal end to the distal end.

15. An electrode lead conveying system, comprising a delivery sheath and the electrode lead of claim 14,
the delivery sheath comprising a sheath entrance, a tubular body and a sheath exit, the tubular body comprising at least one bend,
the delivery sheath configured to deliver the electrode lead to a target position within a target organic tissue.
